(19) Europäisches Patentamt
European Patent Office
Office européen des brevets

(11) **EP 4 647 509 A1**

(12) **EUROPEAN PATENT APPLICATION**
published in accordance with Art. 153(4) EPC

(43) Date of publication:
**12.11.2025 Bulletin 2025/46**

(21) Application number: **24752810.2**

(22) Date of filing: **02.02.2024**

(51) International Patent Classification (IPC):
*C12Q 1/6874* (2018.01)    *C12Q 1/6806* (2018.01)
*C40B 50/06* (2006.01)

(52) Cooperative Patent Classification (CPC):
**C12Q 1/6806; C12Q 1/6874; C40B 50/06**

(86) International application number:
**PCT/CN2024/075583**

(87) International publication number:
**WO 2024/164955 (15.08.2024 Gazette 2024/33)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.02.2023 CN 202310133873**

(71) Applicant: **GeneMind Biosciences Company Limited**
**Shenzhen, Guangdong 518023 (CN)**

(72) Inventor: **LI, Linsen**
**Shenzhen, Guangdong 518023 (CN)**

(74) Representative: **Michalski Hüttermann & Partner Patentanwälte mbB**
**Kaistraße 16A**
**40221 Düsseldorf (DE)**

(54) **SEQUENCING METHOD**

(57)    Provided is a sequencing method. The sequencing method comprises: providing a solid support, wherein the solid support is provided with a first site, a plurality of nucleic acid molecules are immobilized at the first site, and the plurality of nucleic acid molecules have n nucleic acid templates having different sequences, and n is a natural number ranging from 2 to 4; implementing multiple cycles of base extension on the nucleic acid molecules, acquiring sequencing signals of channels in a base extension process. and in one or more cycles of base extension reaction, sequencing signals at the first site at least including a first sequencing signal having the highest signal intensity and a second sequencing signal having the second-highest signal intensity; and on the basis of the intensity difference of the sequencing signals of the channels, classifying the sequencing signals, and determining sequencing results of the at least two nucleic acid templates. According to the embodiments, by using the sequencing method, sequencing signals can be effectively classified by means of the intensity difference, thereby fully utilizing a multi-molecule signal site, and improving the sequencing throughput.

FIG. 2

## Description

## FIELD

The present disclosure relates to the technical field of sequencing, in particular to a sequencing method.

## BACKGROUND

Next-generation sequencing (NGS), also known as high-throughput sequencing or massively parallel sequencing, is initially developed based on the principle of pyrosequencing. Adopting sequencing-by-synthesis (SBS) as its fundamental design concept, the pyrosequencing uses a target nucleic acid sequence (nucleic acid to be tested ) as a template, and introduces specific nucleotides through a series of base extension reactions, so that the sequence of the target nucleic acid sequences may be determined based on the types of the nucleotides introduced.

High-throughput sequencing enables simultaneous detection of numerous target genes and their variant sites in a single detection. It delivers high detection sensitivity and specificity while supporting both qualitative and quantitative detection. Moreover, its cost for testing equivalent quantities of genes and sites remains relatively low. Therefore, High-throughput sequencing has demonstrated extremely broad clinical and research application across many fields, such as noninvasive prenatal screening (NIPS), tumor gene mutation, genetic disease, preimplantation genetic screening (PGS), preimplantation genetic diagnosis (PGD), pathogen, and metagenomics, and has become the most efficient tool for DNA and RNA sequence analysis, and also serves as a cornerstone technology for research, and clinical disease diagnosis and treatment in the era of precision medicine.

However, current sequencing-by-synthesis technologies still require improvement. For example, the current sequencing method fails to perform an effective sequencing on a position containing two or more different nucleic acid templates, such as a position containing two different nucleic acid molecule clusters. Specifically, during each cycle of sequencing reaction, such a position containing two or more different sequencing templates simultaneously generates sequencing signals, which are superimposed and thus cannot be effectively distinguished, resulting to an inefficient utilization of the sequencing data at the position, thereby resulting to a failed sequencing at the position and decrease of sequencing throughput.

## SUMMARY

The present disclosure aims at solving one of the above technical problems at least to some extent or at least providing a useful commercial option. Therefore, an object of the present disclosure is to provide a sequencing method capable of increasing the sequencing throughput, enabling simultaneous sequencing of different nucleic acid templates within one position.

The present disclosure is accomplished based on the following findings by the inventors:
In nucleic acid sequencing, technicians usually strive to ensure that only one type of base signal exists in the same position, i.e., signal bright spot (or signal spot, or bright speckle) on the solid support. If two or more base signals coexist in one bright spot during the same cycle of base extension, such a position is generally referred to as a multi-molecule signal spot. Multiple sequencing signals generated by such a multi-molecule signal spot cannot be effectively distinguished, interfering with base calling and thereby decreasing accuracy. Therefore, in practice, the conventional operation involves discarding sequencing signals and corresponding sequencing data from the multi-molecule signal spot. However, this operation significantly reduces the effective utilization of sequencing signals, thereby decreasing the throughput, because admixing by a few other nucleic acid molecules at the positions on the solid support is difficult to eliminate entirely. Based on in-depth research and experience in nucleic acid sequencing, the inventors of the present disclosure have creatively proposed a sequencing method capable of utilizing sequencing signals generated by the multi-molecule signal spot to increase the sequencing throughput. For the same position, i.e., signal bright spot, the inventors of the present invention perform a classification on the multiple sequencing signals generated by the multi-molecule signal spot based on differences in signal intensities.

Therefore, a first aspect of the present disclosure proposes a sequencing method. According to embodiments of the present disclosure, the sequencing method includes: providing a solid support, wherein the solid support is provided with a first position, at which a plurality of nucleic acid molecules are immobilized, the plurality of nucleic acid molecules having n different nucleic acid templates with different sequences, wherein n is an integer from 2 to 4; subjecting the nucleic acid molecules to multiple cycles of base extension and acquiring sequencing signals from respective channels during the base extension, wherein in the one or more cycles of base extension, the sequencing signals at the first position include at least a first sequencing signal with the highest signal intensity and a second sequencing signal with the second-highest signal intensity; and assigning the sequencing signals and determining a sequencing result of at least two types of nucleic acid templates, based on differences in the signal intensities of the respective sequencing signals.

Therefore, according to embodiments of the present disclosure, the present invention enables multi-molecule signals to be identified effectively. On encountering such a multi-molecule signal, multiple bases may be identified based on signal characteristics, and finally, sequences of two or more sequencing reads may be identified from a signal spot in images of multiple cycles of sequencing. According to embodiments of the present disclosure, with the use of the method, sequencing signals may be classified by performing analysis on the signal intensity differences among a plurality of sequencing signals at the first position, thereby effectively achieving full utilization of the multi-molecule signals, and significantly increasing the sequencing throughput. Moreover, by employing the method, the loss of important information from a nucleic acid template with lower molecule numbers at the first position may also be prevented, enabling the true sequence of the target subject to be reflected more accurately by the sequencing result.

The additional aspects and advantages of the present disclosure will be partially given in the following description, and some will be apparent according to the following description, or be understood through the practice of the present disclosure.

## BRIEF DESCRIPTION OF THE DRAWINGS

The above and/or additional aspects and advantages of the present disclosure will be apparent and readily appreciated from the description of embodiments taken in conjunction with the following drawings, in which:

FIG. 1 shows a schematic diagram of conventional sequencing;
FIG. 2 shows a flowchart of a sequencing method according to an embodiment of the present disclosure; and
FIG. 3 shows a statistical diagram of sequencing data provided by embodiment 1 of the present disclosure.

## DETAILED DESCRIPTION

Reference will be made in further detail to the present disclosure in combination with embodiments, to make the technical problems to be solved, the technical solutions and beneficial effects of the present disclosure clearer. It should be understood that the specific embodiments described herein are only used to explain the present disclosure, rather than limit the present disclosure.

The term "at least one" refers to one or more, and "plurality (multiple)" means two or more. "At least one" or expressions similar thereto refers to any combination of these items, including any combination of a single item or a plurality of items. For example, "at least one of a, b, or c" or "at least one of a, b, and c" may mean: a, b, c, a-b (i.e., a and b), a-c, b-c, or a-b-c, where a, b, and c may be single or multiple terms, respectively.

As used in the embodiments of the present disclosure and the appended claims, the singular forms of "a," "an," "the," and "said" are intended to include the plural forms unless the context clearly indicates otherwise.

It should be understood that in various embodiments of the present disclosure, the sequence number of each process does not mean the execution sequence, where some or all of the steps may be executed in parallel or sequentially, and the execution sequence of each process should be determined by its function and internal logic, and should not constitute any limitation on the implementation process of the embodiments of the present disclosure.

The terms "first", "second", "third", and "fourth" are used herein merely for purposes of description, to distinguish objectives such as locations, objects, etc. from each other, and shall not be understood as indicating or implying relative importance or implicitly indicating the number of technical features indicated. For example, without departing from the scope of the embodiments of the present disclosure, "first" and "second" may be interchanged, such that a first position may also be referred to as a second position, and correspondingly, the second position may also be referred to as the first position. Thus, features defined by "first", "second", "third", and "fourth" may explicitly or implicitly include one or more of such features.

In embodiments of the present disclosure, the term "sequencing" may also be referred to as "nucleic acid sequencing" or "gene sequencing", which may be interchangeable in terms of expression, and all refer to the determination of the type and arrangement order of bases or nucleotides, including nucleotide analogs, in nucleic acid molecules. Sequencing as it is called involves the process of nucleotides binding to a template and collecting the corresponding sequencing signals generated by the nucleotides, including analogs. Sequencing involves sequencing by synthesis (SBS) and/or sequencing by ligation (SBL), including DNA sequencing and/or RNA sequencing, and long fragment sequencing and/or short fragment sequencing. Long fragments and short fragments as they are called are relative, for example, nucleic acid molecules longer than 1 Kb, 2 Kb, 5 Kb or 10 Kb may be referred to as long fragments, while nucleic acid molecules shorter than 1 Kb or 800 bp may be called short fragments.

The sequencing generally involves multiple cycles of process to achieve the determination of the types and order of multiple bases or nucleotides on the nucleic acid template. The examples of the present application refer to each cycle of the "process to achieve the determination of the types and sequence of multiple bases or nucleotides on the nucleic acid template" as one "cycle of sequencing". The "cycle of sequencing", also known as "sequencing cycle, may be defined as the completion of one base extension of the four types of nucleotides/bases; in other words, one "cycle of sequencing" may be defined as the determination of the base or nucleotide type at any given position on the template. For sequencing platforms that achieve sequencing on the basis of polymerization or ligation reactions, one cycle of sequencing includes the process of binding four types of nucleotides (including nucleotide analogs) to the nucleic acid template at a time according to the base complementary rule and acquiring the corresponding signals emitted. For platforms that achieve sequencing on the basis of the polymerization reaction, a reaction system includes reaction substrate nucleotides, polymerase, and a nucleic acid template. A sequence fragment (a sequencing primer) binds to the nucleic acid template, and on the basis of the base pairing rules and the principle of polymerization reaction, the added reaction substrate nucleotides are linked to the sequencing primer under the catalysis of the polymerase to achieve the binding of the nucleotide to a specific position on the nucleic acid template. Generally, one cycle of sequencing may include one or more base extensions (repeats). For example, four types of nucleotides are sequentially added to the reaction system to each perform base extensions and corresponding acquisition of reaction signals, and one cycle of sequencing includes four base extensions; for another example, four types of nucleotides are added into the reaction system in any combinations (such as in pairs or in one-three combinations), the two combinations each perform base extensions and corresponding acquisition of reaction signals, and one cycle of sequencing includes two base extensions; for yet another example, four types of nucleotides are added simultaneously to the reaction system for base extension and reaction signal acquisition, and one cycle of sequencing includes one base extension.

The term "nucleic acid molecule" refers to a polymeric form of nucleotides of any length, and may include ribonucleotides or analogs thereof, deoxyribonucleotides or analogs thereof, and mixtures thereof. The nucleic acid molecule may be referred to as a single-stranded polynucleotide or a double-stranded polynucleotide. Nucleotides in a nucleic acid molecule may include naturally occurring nucleotides and functionally alternative analogs thereof. Examples of the analogs may hybridize to the nucleic acid in a sequence-specific manner, or may be used as templates for replication of a specific nucleotide sequence. Naturally occurring nucleotides typically have a backbone including phosphate diester bonds. The analog may be of a structure with alternative backbone linkage including any of a variety of linkage known in the art. Naturally occurring nucleotides typically have deoxyribose (e.g., as found in DNA) or ribose (e.g., as found in RNA). The analog may be of a structure with substituted sugar moieties, including any of those known in the art. Nucleotides may include natural bases or non-natural bases. Bases in natural DNA may include one or more of adenine, thymine, cytosine, and/or guanine, and bases of the natural RNA may include one or more of adenine, uracil, cytosine, and/or guanine. Nucleotides may also include any non-natural base or analog thereof, such as locked nucleic acids (LNAs) and bridging nucleic acids (BNAs).

The term "nucleic acid template" refers to an original nucleic acid molecule or a fragment thereof that is bound with nucleotides or nucleotide analogs by successive cycles of base extension. The nucleic acid template may be the entire sequence of a nucleic acid molecule or a partial fragment thereof. The nucleic acid template may be a nucleic acid fragment that is used as a template in an extension reaction during sequencing by synthesis, where since the bases added in the extension reaction satisfy the base-pairing principle with the sequencing template, the sequence of the sequencing template could be determined by detecting the type of bases added in each cycle of extension. The sequence of the nucleic acid template herein may generally include, but is not limited to, at least one of a sequence of a target molecule, a UMI sequence, a sample barcode sequence, and is sometimes referred to herein as an "insert fragment" or a "target molecule".

The term "cluster" refers to a plurality of nucleic acid molecules generally included in one signal spot on a solid support, such as a chip, but usually the plurality of nucleic acid molecules at this signal spot are derived from the same nucleic acid molecule, for example, an amplified cluster including nucleic acid molecules formed by performing bridge PCR on the same nucleic acid molecule.

The term "primary nucleic acid molecule" as used herein refers to the nucleic acid molecule with the highest copy number in a signal spot or a position. The term "secondary nucleic acid molecule" as used herein refers to a nucleic acid molecule with a copy number lower than that of the primary nucleic acid molecule in a signal spot or a position. The reason of generation of the secondary nucleic acid molecule is not particularly limited. It may be generated during bridge amplification or may result from the binding of a plurality of different nucleic acid molecules at the same position during amplification of the nucleic acid molecules at the signal spot.

The term "primer", also referred to as "probe", refers to an oligonucleotide or nucleic acid molecule that may hybridize to a target sequence of interest. In embodiments, the primer functions as a substrate onto which nucleotides may be polymerized by polymerase. For example, primers may be used as starting points for DNA or RNA synthesis. For example, a sequencing primer may hybridize to a synthesized nucleic acid template strand, so as to initiate a synthesis of a new strand complementary to the synthesized nucleic acid template strand. Primers may include any combination of nucleotides or analogs thereof. In some examples, the primer is a single-stranded oligonucleotide or polynucleotide.

The term "crosstalk", "laser-crosstalk" or "spectra-crosstalk", also known as "spectral crosstalk" or "spectral crossover" in embodiments of the present disclosure refers to a signal corresponding to one base diffusing into a signal corresponding to another base. For sequencing platforms that use differently labeled fluorescent molecules to identify different bases, if emission spectra of two or more selected fluorescent molecules overlap, it is possible to detect signal diffusion of one fluorescent molecule into another fluorescent channel in one cycle of sequencing.

The term "phasing", also known as "phase imbalance", "dephasing", or "phase difference", refers to the phenomenon in chemical reactions where reactions among a group, such as nucleic acid molecules in a nucleic acid molecule cluster, are not synchronized, including phasing or sequence lag, and prephasing or sequence lead.

The term "channel" in embodiments of the present disclosure refers to an optical channel formed by using different excitation lights, different fluorescence color filters, etc. in the photographing during sequencing, which are capable of screening and distinguishing fluorescence signals of four fluorescent bases from A, C, G and T/U (indicating T or U). In actual sequencing, photographs are taken in four different fluorescence channels. Ideally, each fluorescence channel only has the fluorescence signal of the corresponding fluorescent base type, but in practice, due to the influence of fluorescence crosstalk, fluorescence signals of other bases will also appear in each channel in addition to the fluorescence signal of the corresponding fluorescent base. The "channel" referred to in embodiments of the present disclosure includes a four-channel condition, , which refers to four optical channels formed by using different excitation lights, different fluorescence color filters, etc. in the photographing during sequencing, which are capable of screening and distinguishing fluorescence signals of four fluorescent bases from A, C, G and T/U (indicating T or U). The "channel" referred to in the embodiment of the present disclosure also includes a dual-channel condition, which refers to optical channels formed by using different excitation lights, different fluorescence color filters, etc., screening and distinguishing fluorescence signals of two fluorescent bases from A, C, G and T/U each time, and obtains, through two times, fluorescence signals of four fluorescent bases A, C, G and T/U. The "channel" referred to in the embodiment of the present disclosure also includes a single-channel condition, which refers to an optical channel formed by using different excitation lights, different fluorescence color filters, etc., screening and distinguishing fluorescence signals of one fluorescent bases from A, C, G and T/U each time, and obtains, through four times, fluorescence signals of four fluorescent bases A, C, G and T/U. Of course, the number of channels is not limited by the above.

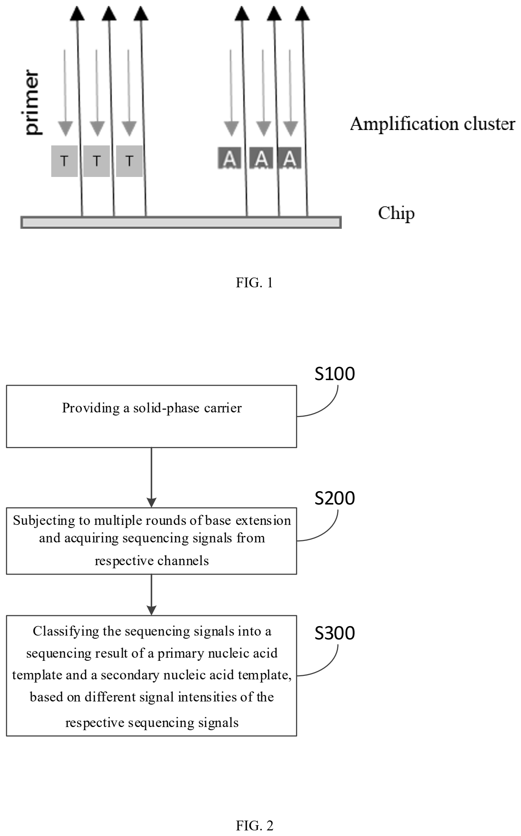

For the purpose of obtaining recognizable signals and improving the data utilizability, the current high-throughput sequencing, when performing signal collection on a position or a nucleic acid molecule cluster, usually strive to ensure that only one type of base signal exists in the bright spot (signal spot) formed by the same position. In other words, as shown in FIG. 1, existing sequencing methods usually subject each cluster to the same base extensions, with the same bases added in each cycle of base extension. In other words, existing base sequencing strives to ensure that only one type of base signal exists in the same signal spot. If two or more base signals coexist in one bright spot in the image collected from one cycle of base extension, such a position or signal spot is generally referred to as a multi-molecule signal spot. The identification of introduced bases may be interfere with the multiple signals, resulting to the decreased identification accuracy. Therefore, the conventional sequencing usually expect that only one type of base signal exists in the each signal spot, i.e. each signal spot corresponds to a single signal, and expect that the presence of these multi-molecule signal spots to be avoided. However, conventional sequencing exhibits certain limitations that restrict its application in scenarios with the presence of multi-molecule signal spots.

For example, during the sequencing process in practice, when library molecules are immobilized on the surface of the solid support, a nucleic acid molecule in the library randomly hybridize with a probe immobilized on the surface of the solid support and is immobilized at a specific location, which referred to as a position. In most cases, one nucleic acid molecule is immobilized per position, and further amplified to form an amplification cluster. However, in occasional cases, a few other nucleic acid molecules may be immobilized at the position, resulting to the amplification clusters formed by two set of nucleic acid templates copies (i.e., forming two different nucleic acid molecule clusters), thereby generating multiple sequencing signals of the nucleic acid templates at the position. Illustratively, when the solid support is a nanowell array chip, the two set of nucleic acid templates copies are formed within one nanowell. In the images collected from sequencing reaction, the signal spot corresponding to this position is associated with two nucleic acid templates. In subsequent data analysis, the two sequencing signals generated at the same position during a cycle of base extension process may not be identified effectively, the data from this position is discarded to decrease the impact of such multi-molecule signal spots on sequencing accuracy. This reduces the effective data size of sequencing, thereby decreasing sequencing throughput.

From this, the inventors of the present invention propose a novel sequencing method, which allows multiple nucleic acid templates with different sequences to coexist at a position simultaneously. In each cycle of extension, same or different bases are independently added to the multiple nucleic acid molecules with different sequences, enabling base calling. For the position containing multiple nucleic acid templates, the method may perform a synchronous sequencing on the multiple nucleic acid templates with different molecule number, so that the signal intensities of sequencing signals generated by nucleic acid templates with different sequences are different to subsequently distinguish and assign the sequencing signals. In other words, the method performs a synchronous sequencing on the multiple nucleic acid templates with different sequences in the position containing multiple nucleic acid templates with different sequences, so that differences in the signal intensities of sequencing signals are generated by at least a primary nucleic acid molecule, hereinafter also referred to as the "first nucleic acid molecule", having a dominant molecule number, and a secondary nucleic acid molecule, hereinafter also referred to as the "second nucleic acid molecule". Accordingly, multiple sequencing signals generated at the same position during a cycle of base extension can be retained, and sequencing data corresponding to these sequencing signals can still be effectively utilized to obtain the sequencing results for at least the primary nucleic acid molecule and the secondary nucleic acid molecule, thereby increasing the sequencing throughput of sequencing reactions. According to embodiments of the present disclosure, this may result from performing an amplification at the indicated position on a nucleic acid molecule from other positions by mistake during nucleic acid library construction, or may result from the a plurality of nucleic acid molecule set at indicated position on purpose.

Specifically, referring to FIG. 2, the sequencing method includes:

S100, providing a solid support.

A solid support is provided in this step. In embodiments of the present disclosure, a solid support is understood as a carrier or support for immobilizing nucleic acid molecules or nucleic acid templates. In some application examples, the solid support may also be referred to as a solid substrate, sequencing chip, or sequencing biochip. In an embodiment, the solid support is a substrate having an array of micropores, and each position corresponds to a position where a micropore is located. An exemplary solid support is a nanowell pattern chip including an array arrangement of nanowells, which may also be referred to as nanopores, micropores or pores. In embodiments of the present disclosure, the nanowell and nanopore referred to herein may be exchangeable for each other, and refer to a pore structure with nanoscale size formed on the surface of a solid support. The shape of the nanowell or nanopore is not strictly limited and may be circular, elliptical, triangular, quadrilateral or other polygonal, or even no particular shape. To facilitate uniformity of nucleic acid molecule amplification, in some embodiments, the nanowell or nanopore is arranged in the shape of a symmetrical pattern in cross section, exemplarily, the cross section of the nanowell or nanopore is circular or regular polygon, and the more sides of the regular polygon, the more favorable it is for smooth extension of the nucleic acid molecule amplification. Examples of pattern chips include pattern chips from Illumina Inc. and DNB (DNA nanoball) pattern chips from MGI. In an embodiment, the solid support is a planar substrate, the surface of which includes a plurality of surface-treated positions, one position corresponding to a position. Examples of such solid support are such as random chips.

In this embodiment, the surface of the solid support is immobilized with nucleic acid molecules, and the nucleic acid molecules are linked to the surface of the solid support. In one embodiment, the surface of the solid support, via a covalent bond to a molecule/group on the surface thereof, is linked with probes, and the nucleic acid template is linked to the surface of the solid support via the probe. The probe is an oligonucleotide or nucleic acid molecule fragment capable of hybridizing to a target nucleic acid molecule of interest. In an embodiment, at least a portion of the probe is configured to hybridize to at least a portion of a 3' end of the nucleic acid template. In another embodiment, one end of the nucleic acid template is covalently bonded to a molecule/group on the surface of the solid support, thereby attaching to the surface of the solid support.

In this embodiment, a plurality of positions are provided on the solid support, and the nucleic acid molecule is immobilized at the surface on a position of the solid support. The position on the surface of the solid support may be understood as a point of the solid support for immobilizing the amplification cluster of nucleic acid molecules, and there is a certain distance between two adjacent such positions, and this distance is sufficiently larger than a distance between nucleic acid molecules in the same position, such that sequencing signals corresponding to different positions, during the collection of sequencing signals generated by the base extensions, can be distinguished on the image. Illustratively, each nanowell is a position on the surface of the nanowell pattern chip. In embodiments of the present disclosure, the solid support may be designed to determine distribution or arrangement of positions. In an embodiment, for a non-nanowell pattern chip, the position may be determined by forming dot probe clusters on the surface of a solid support. Similarly, a probe cluster refers to a plurality of probes clustered within a single point on the surface of a solid support, and there is a certain distance between two adjacent probe clusters, and this distance is sufficiently greater than a distance between probes in the same probe cluster, such that signals generated at the positions where different probe clusters are located could be distinguished when base extensions are performed on nucleic acid molecules to which probes are linked.

In embodiments of the present disclosure, a plurality of nucleic acid molecules are immobilized on the positions of the solid support, ideally, one nucleic acid is immobilized in a hybridization manner at each position. In some unavoidable cases, there may be conditions where there are very few positions with no nucleic acid molecules immobilized or only one nucleic acid molecule immobilized at one position.

Conventionally, in the sequencing signals obtained by one cycle of base extension, two or more different sequencing signals appearing at one position indicates that there may be two or more nucleic acid molecules with different sequences. In order to improve sequencing accuracy, sequencing signals at these positions will be not counted, and the corresponding sequencing data will be removed. Different from the expectation of sequencing only one nucleic acid template set per position in the past, in embodiments of the present disclosure, two or more different nucleic acid templates with different sequences are allowed to coexist at some positions, and the sequencing method provided by embodiments of the present disclosure includes sequencing different nucleic acid templates in these positions. Because the position containing two or more nucleic acid templates with different sequences will generate two or more signals in one cycle of base extensions, this corresponding position may also be called a multi-molecule position.

For the solid support provided in the embodiments of the present disclosure, positions on the surface of the solid surpport may be classified into several types based on the types of nucleic acid templates with different sequences contained at the position. In the embodiments of the present disclosure, the positions include one or more first positions. A plurality of nucleic acid molecules are immobilized per position, and has n types of nucleic acid templates with different sequences, where n is an integer from 2 to 4. Among these, the nucleic acid templates at the first position of the surface include a target nucleic acid molecule derived from the construction of a sample library, and may also include an additional nucleic acid molecule as impurity introduced prior to base extension. It should be understood that at the position, the molecule number of additional nucleic acid molecule as impurity is significantly lower than that of the target nucleic acid molecule, which generate a low signal intensity of the sequencing signal, thereby forming background noise or being easily submerged within background noise. In an embodiment of the present disclosure, the n types of nucleic acid templates with different sequences refer to the target nucleic acid molecule.

In an embodiment of the present disclosure, the molecule numbers of the n types of nucleic acid templates with different sequences at the first position are different from each other. Correspondingly, n types of sequencing signals with different intensities are generated at the first position during each cycle of base extension. The n types of nucleic acid templates with different sequences at least include a first nucleic acid molecule with the highest molecule number and a second nucleic acid molecule with the second-highest molecule number. In some embodiments, the first position is provided with two types of nucleic acid templates with different sequences and different molecule numbers, namely the first nucleic acid molecule and the second nucleic acid molecule, also referred to as the primary nucleic acid molecule and the secondary nucleic acid molecule. Correspondingly, the sequencing signals include a first sequencing signal with the highest signal intensity and a second sequencing signal with the second-highest signal intensity. In some embodiments, the first position is provided with three types of nucleic acid templates with different sequences and different molecule numbers, namely a first nucleic acid molecule, a second nucleic acid molecule, and a third nucleic acid molecule. Correspondingly, the sequencing signals include a first sequencing signal, a second sequencing signal, and a third sequencing signal with sequentially decreasing signal intensities. In some embodiments, the first position is provided with four types of nucleic acid templates with different sequences and different molecule numbers, namely a first nucleic acid molecule, a second nucleic acid molecule, a third nucleic acid molecule, and a fourth nucleic acid molecule. Correspondingly, the sequencing signals include a first sequencing signal, a second sequencing signal, a third sequencing signal, and a fourth sequencing signal with sequentially decreasing signal intensities. In the embodiments of the present disclosure, the terms "first", "second", "third", and "fourth" are used merely for purposes of distinguishing the types of nucleic acid templates with different sequences based on the molecule numbers of the nucleic acid molecules at the first position.

In an embodiment of the present disclosure, when the n types of nucleic acid templates with different sequences at the first position have an approximately equal number of molecules, the signal intensities of the two sequencing signals generated by the first position during each cycle of base extension are nearly the same. In the case of the sequences of the n types of nucleic acid templates are unknown, the sequencing signals from each cycle of base extension cannot be assigned to different nucleic acid templates, making it impossible to distinguish the sequence of the two nucleic acid molecules. Therefore, in an embodiment of the present disclosure, the n types of nucleic acid templates with different sequences at the first position are different from each other in molecule numbers. Usually, the greater the difference in molecule numbers between the n types of nucleic acid templates with different sequences, the more significant the difference in the signal intensities of the sequencing signals they generate, contributing to determining the sequences of the nucleic acid molecules by connecting in series the sequencing signals generated during each cycle of base extension subsequently according to the signal differences. In some embodiments, at least two in the n types of nucleic acid templates with different sequences generate different sequencing signals in at least one cycle of base extension.

In some embodiments, at the first position, for any two sequencing signals whose molecule numbers are adjacent in magnitude, the molecule number of the nucleic acid template with a higher molecule number is at least 1.2 times the molecule number of the nucleic acid template with a lower molecule number between two nucleic acid templates adjacent in molecule number, among the nucleic acid templates ranking in a descending or ascending order. In other words, at the same first position, the intensity of the sequencing signal generated by the nucleic acid molecule cluster with a higher molecule number is approximately at least 1.2 times the intensity of the sequencing signal generated by the nucleic acid molecule cluster with a lower molecule number. In other words, for any two sequencing signals whose intensities are adjacent in magnitude, the signal intensity of a higher intensity signal is at least 1.2 times the signal intensity of a lower intensity signal among the sequencing signals ranking in a descending or ascending order of signal intensities. Accordingly, during a cycle of base extension, the different intensities of the sequencing signals generated by different nucleic acid templates at the same first position are exhibited in an equivalent proportion to the ratio of nucleic acid molecule numbers, contributing to being distinguished between them during a cycle of base extension. Consequently, the efficiency of assigning the sequencing signals based on intensity differences can be further increased, thereby further increasing sequencing efficiency. It should be understood that, when the difference in molecule numbers between two nucleic acid templates with different sequences reaches a certain level, the sequencing signal generated by the nucleic acid template with lower molecule number may be masked or become to an indistinct background signal unrecognizable. Therefore, in the embodiment of the present disclosure, at the first position, the molecule number of the nucleic acid template with the higher molecule number is at most 10 times the molecule number of the nucleic acid template with the lower molecule number.

In some embodiments, for any two sequencing signals whose molecule numbers are adjacent in magnitude, the molecule number of the nucleic acid template with a higher molecule number is at least 1.5 times the molecule number of the nucleic acid template with a lower molecule number between two nucleic acid templates adjacent in molecule number, among the nucleic acid templates ranking in a descending or ascending order. In this case, at the same first position, the intensity of the sequencing signal generated by the nucleic acid molecule cluster with a higher molecule number is approximately at least 1.5 times the intensity of the sequencing signal generated by the nucleic acid molecule cluster with a lower molecule number, in one cycle of base extension. In other words, for any two sequencing signals whose intensities are adjacent in magnitude, the signal intensity of a higher intensity signal is at least 1.5 times the signal intensity of a lower intensity signal among the sequencing signals ranking in a descending or ascending order of signal intensities. Both exhibit a relatively significant difference in sequencing signal intensity, thereby contributing to identification of the sequencing signal with high intensity and the sequencing signal with low intensity among the sequencing signals generated during each cycle of base extension. During respective cycles of base extension, the sequencing signal with high intensity is derived from the nucleic acid molecule with a higher molecule number (e.g., the primary nucleic acid molecule), while the sequencing signal with low intensity is derived from the nucleic acid molecule with a lower molecule number (e.g., the secondary nucleic acid molecule). Therefore, the base calling result for the nucleic acid template with a higher molecule number can be obtained by connecting in series the base calling results corresponding to the sequencing signals with higher intensity obtained from respective cycle of base extension, and the base calling result for the nucleic acid template with a lower molecule number can be obtained by connecting in series the base calling results corresponding to the sequencing signals with lower intensity obtained from respective cycle of base extension.

In some embodiments, for any two sequencing signals whose molecule numbers are adjacent in magnitude, the molecule number of the nucleic acid template with a higher molecule number is at least 1.5 to 10 times the molecule number of the nucleic acid template with a lower molecule number between two nucleic acid templates adjacent in molecule number, among the nucleic acid templates ranking in a descending or ascending order. Correspondingly, for any two sequencing signals whose intensities are adjacent in magnitude, the signal intensity of a higher intensity signal is at least 1.5 to 10 times, the signal intensity of a lower intensity signal among the sequencing signals ranking in a descending or ascending order of signal intensities. Therefore, in a cycle of base extension, different nucleic acid templates exhibit a relatively significant difference in sequencing signal intensity. Then the sequencing signal generated by the nucleic acid template with lower molecule number still maintains a certain intensity relative to the nucleic acid template with higher molecule number, not being masked or becoming to an indistinct background signal unrecognizable.

It should be understood that, in an embodiment of the present disclosure, the absolute molecule numbers of the n types of nucleic acid templates with different sequences at the first position may not be calculable by effective means, however, the relative molecule numbers between different nucleic acid templates may be obtained by comparison. For example, the ratio or proportion of relative molecule numbers of different nucleic acid templates is obtained through the signal intensities of sequencing signals collected from one or more cycles of base extension. Similarly, the ratio or multiple of molecule numbers described in the embodiment of the present disclosure is also the ratio or multiple based on the relative molecule numbers. Specifically, the relative molecule numbers of nucleic acid templates with different sequences at this first position are determined through sequencing signals and the signal intensities thereof collected from respective channels during one or more sequencing reaction processes. In an embodiment, at the same first position, the nucleic acid templates with different sequences generate different sequencing signals in at least one cycle of base extension. At this point, the relative number or relative content of the different nucleic acid templates at this first position may be determined through the signal intensities of sequencing signals generated by the indicated base extension. In another embodiment, at the same first position, the nucleic acid templates with different sequences may generate partially identical sequencing signals in a cycle of base extension. At this point, by a cycle of sequencing reaction, the relative number or relative content of the nucleic acid molecules with different sequencing signals at this first position may be determined. The nucleic acid molecules whose relative number or relative content remains undetermined, will also generate different sequencing signals during other cycles of base extension, and their relative number or relative content will be determined by identifying the signal intensities of the generated sequencing signals based on the same approach at this point. Two or more such cycles of base extension are performed until the relative number or relative content of nucleic acid templates with different sequences at the same first position is determined. On this basis, the type of a base or nucleotide introduced for respective nucleic acid templates during the current cycle of sequencing reaction may be determined based on the relative number or relative content of respective nucleic acid templates at this first position and the signal intensities of sequencing signals generated by respective cycles of sequencing reaction. Even if two nucleic acid templates with different sequences generate identical sequencing signals, the sequencing signals may be assigned based on the immobilized relative number or relative content of respective nucleic acid molecules. In other words, for a first position containing a plurality of nucleic acid molecules with different sequences, the type of a base or nucleotide introduced during respective cycles of base extension may be identified according to signal characteristics. Then, a plurality of sequences of different nucleic acid molecules may be identified from the first position, in sequencing signals, such as a collected fluorescence image, acquired by multiple cycles of base extension.

For example, a same first position on an array chip containing two nucleic acid molecules with different sequences is exemplarily illustrated. In principle, there may be dual-molecule signal position formed by amplification clusters of two nucleic acid templates with different sequences in a nanowell of the array chip. Due to differences in amplification initiation timing and amplification efficiency, the ratio of amplification number between these two nucleic acid molecules is 7:3. In each cycle of base extension, the signal intensities generated by the two nucleic acid molecules in two specific base channels also is exhibited in a ratio of 7:3. Based on the intensity characteristics of the sequencing signals obtained from each cycle of base extension, the type of the base or nucleotide introduced by the two nucleic acid templates during each base extension may be ascertained.

Among the n types of nucleic acid templates with different sequences, a larger value of n enables a position to identify more nucleic acid molecules. However, limited by the number of base types or base channels, a position can achieve base calling for at most four nucleic acid templates with different sequences. In this case, the types of nucleic acid molecules at the same first position are controlled within 4.

In some embodiments, the solid support is further provided with a second position, at which a plurality of nucleic acid molecules are immobilized, the plurality of nucleic acid molecules having identical sequences. In other words, the nucleic acid molecules at the second position are nucleic acid molecules with identical sequences.

According to embodiments provided in the present disclosure, the solid support is provided with a plurality of positions. A plurality of nucleic acid molecules are immobilized on the plurality of positions, respectively. Prior to fixing the plurality of nucleic acid molecules on the positions, the method includes constructing sequencing library molecules based on a target subject. The plurality of nucleic acid molecules immobilized at the plurality of positions respectively are obtained by performing amplification on the sequencing library molecules on the surface of the solid support.

According to embodiments of the present disclosure, the plurality of nucleic acid molecules are immobilized on the surface of the positions through bridge amplification.

According to embodiments of the present disclosure, the plurality of nucleic acid molecules at the first position are formed by performing bridge amplification on different nucleic acid templates. In other words, the n types of nucleic acid templates with different sequences are formed by performing bridge amplification on different templates.

S20, subjecting the nucleic acid molecules to multiple cycles of base extension and acquiring sequencing signals from respective channels during the base extension.

In this step, multiple cycles of base extension are performed on the nucleic acid molecules, and sequencing signals from respective channels are acquired during the base extension. In embodiments of the present disclosure, during one or more cycles of base extension, the sequencing signals at the first position include at least the first sequencing signal with the highest signal intensity and the second sequencing signal with the second-highest signal intensity.

After obtaining the sequencing chip, multiple cycles of sequencing reactions are performed on the sequencing chip, and sequencing signals from respective base channels are acquired for a given position during the sequencing reactions. According to embodiments of the present disclosure, base extension may be implemented through sequencing-by-synthesis reactions or sequencing-by-ligation.

During sequencing reactions, each nucleic acid template expands one nucleotide (or nucleotide analog) linked to a specific signal group (typically a fluorophore) in each cycle of base extension. By detecting the sequencing signal (typically a fluorescence signal) generated by the specific signal group in a specific channel, the type of introduced base or nucleotide may be determined, thereby determining the sequence of the nucleic acid template. In some embodiments, based on the type of fluorophore on the surface of the nucleotide or nucleotide analog introduced in each sequencing cycle, base extension may be classified as base extension based on four different fluorophores (also known as four-color sequencing) and or base extension based on two different fluorophores (also known as two-color sequencing). Correspondingly, the step of acquiring sequencing signals from respective channels includes two scenarios: for the base extension based on four different fluorophores, sequencing signals from four base channels are acquired simultaneously in one acquisition, with each base channel corresponding to a base type; for the base extension based on two different fluorophores, sequencing signals from two base channels are acquired separately twice, ultimately obtaining sequencing signals for all four bases. In two-color sequencing, bases A, T/U, C, and G are classified using bright/dark combinations of 11, 10, 01, and 00 across the two channels, where "1" = bright, the two channels are Green and Red. The resolution for multiple molecules is better in four-color sequencing than two-color sequencing. In four-color sequencing, the classification encoding for the four bases is 1000, 0100, 0010, 0001, exhibiting significantly higher information encoding redundancy than two-color sequencing. In practice, brightness differences, besides bright/dark encoding, are further considered, but obviously, complex base signals can be distinguished better in four-color sequencing than two-color sequencing. Precisely because four-color sequencing can identify multi-signals through intensity differences, it has clear advantages for complex base calling. Thus, the base calling method provided in embodiments of the present disclosure is more suitable for four-color sequencing.

According to embodiments of the present disclosure, at least two nucleic acid templates with different sequences may exist at the first position, correspondingly forming at least two different nucleic acid molecules. These two different nucleic acid molecules are the first nucleic acid molecule and second nucleic acid molecule, also referred as the primary nucleic acid molecule and secondary nucleic acid molecule. It should be understood that in embodiments of the present disclosure, these different nucleic acid molecules at the first position may be either "accidentally" generated at the same first position or "intentionally" generated at the same first position, provided their sequencing signals generated during base extension exhibit sufficient intensity differences to enable to be distinguished. At least a part of the plurality of different nucleic acid molecules may generate different sequencing signals in at least one cycle of sequencing reaction.

In embodiments of the present disclosure, acquiring sequencing signals from respective channels during the base extension includes acquiring sequencing signals from respective channel during each cycle of base extension. According to embodiments of the present disclosure, the molecule numbers of the n types of nucleic acid templates with different sequences are different, correspondingly, the signal intensities of sequencing signals generated by the n types of nucleic acid templates with different sequences are different in each cycle of base extension.

In some embodiments, the sequencing signal is an optical signal. Exemplarily, the sequencing signal is a fluorescence signal generated by the fluorophore on the nucleotide. In this case, acquiring sequencing signals from respective channels includes: after performing one cycle of base extension, collecting fluorescence images from four base channels, and acquiring bright spot signals in the fluorescence images of respective channels. A bright spot in the fluorescence image corresponds to the signal from a position. Correspondingly, signals generated by multi-molecule signal spots are multi-signals, and the corresponding bright spot is a multi-signal spot formed by mixed signals. In some embodiments, acquiring bright spot signals in the fluorescence images of respective channels includes a process of determining the fluorescence intensity of respective bright spots in the fluorescence images. Specifically, the fluorescence intensity of respective bright spots in the fluorescence images may be confirmed, by extracting grayscale values of the fluorescence images and performing correction on the obtained grayscale images. In other words, the signal intensity of the sequencing signal are a corrected signal intensity of the sequencing signal. Exemplarily, the correction includes inter-channel crosstalk correction (e.g., crosstalk) and/or intra-channel crosstalk correction.

To eliminate crosstalk between respective channels, crosstalk correction is performed. Its primary purpose is to ensure that respective fluorophores displays signals only within a single channel, facilitating the identification of true base signals within a position. According to embodiments of the present disclosure, pairwise correction of 4-dimensional data based on permutation and combination yields 12 types of combinations. For example, AC correction denotes that C is used to correct A; AT correction denotes that correction from A to T; TA correction denotes correction from T to A. In an embodiment, the correction process between two channels is performed as: selecting a combination of (x,y) for specific combinations to calculate the crosstalk correction from x to y ; selecting a immobilized-brightness segment in x; for (x,y) pairs within this interval, extracting the lower edge of the (x,y) scatter plot at fixed step size x.

The discrete points obtained in this way characterize the minimal crosstalk from x to y.

Subsequently, linear fitting is performed on the sampled set of discrete points to obtain the linear fitting slope, so that the linear fitting slope k is used for correction calculations, e.g. TA correction, using A to correct T as follows:

$$T^{.} = T - A \times k$$

According to some specific embodiments, it can be seen that after correction, the angle between the A-arm and the horizontal axis in the AT plot becomes zero.

Additionally, according to embodiments of the present disclosure, as the extension progress over multiple cycles of reaction, the inventors observed that the signals of each base is not t purely singular any more. This arises from lead and lag effects in chemical reaction causing phase errors, which may usually be referred as phasing, where bases intended for cycle N react in cycle N+1, and prephasing, where bases intended for cycle N react in cycle N-1. This may be understood simple as the intra-channel crosstalk between adjacent extension reactions in the same channel. Differing from the fixed inter-channel crosstalk, the intra-channel crosstalk accumulates progressively with the development of extension. According to embodiments of the present disclosure, phasing/prephasing correction and crosstalk correction basically consist of three steps, i.e., sampling, linear fitting, and correction. According to embodiments of the present disclosure, inter-channel crosstalk correction is performed prior to intra-channel correction, enabling the lowest error rate under current conditions.

S30. classifying the sequencing signals into a sequencing result of a primary nucleic acid template and a secondary nucleic acid template, based on differences in the signal intensities of the respective sequencing signals.

In this step, the sequencing signals are assigned and the sequencing result of at least two types of nucleic acid templates are determined, based on differences in the signal intensities of the respective sequencing signals.

After obtaining respective sequencing signals, the sequencing signals are classified into the sequencing results of the different nucleic acid templates based on the different intensities of the respective sequencing signals. In general, for a given sequencing reaction, only the sequencing signal with the highest signal will be selected for determining the result of the nucleic acid template, e.g., when there is a significant signal difference between the sequencing signals obtained from respective channels and there is a clear dominant advantage for the highest sequencing signal, the type of the base corresponding to the channel with the highest sequencing signal will be determined as the base that is introduced for the nucleic acid molecule in the current cycle of base extension, and the sequencing signals of other channels will be discarded. In some cases, to avoid the impact of interference between multi-channel sequencing signals on base calling results, even all data from that the signal site is discarded, resulting to the data loss at some positions and decrease of sequencing throughput. However, the inventors of the present invention propose that, based on differences in the signal intensities of the sequencing signals from respective channels, sequencing signals between nucleic acid templates with different sequences at the same first position may be assigned, thereby obtaining sequencing results for nucleic acid templates with different sequences. Thus, the waste of sequencing data can be reduced and the sequencing throughput can be increased.

According to embodiments of the present disclosure, during synchronous sequencing of the first position, because molecule numbers of the nucleic acid templates with different sequences are different, sequencing signals generated by the nucleic acid templates with different sequences are different in signal intensities. Therefore, when determining the sequencing result of the nucleic acid templates, the sequencing results of the a plurality of nucleic acid templates with different sequences may be determined based on differences in the signal intensities of the sequencing signals, by acquiring sequencing signal data of the position.

According to embodiments of the present disclosure, assigning the sequencing signals and determining the sequencing result of at least two types of nucleic acid templates, based on differences in the signal intensities of the respective sequencing signals, include:

(1) determining a number of the nucleic acid templates and relative signal intensities corresponding to the respective nucleic acid templates, based on the types and intensities of the respective sequencing signals acquired from the first position during at least one cycle of base extension.

This step includes a process of aligning signals generated by base extension according to the position of the first position (which can also be regarded as the coordinates of the first position). For the same first position, the sequencing signals from the respective channels at this first position during each cycle of base extension include multiple sequencing signals with different intensities. In an embodiment, the sequencing signal is an optical signal, exemplarily, such as a fluorescence signal.

It should be understood that in this embodiment, prior to the sequencing, it is necessary to determine the order for the number of nucleic acid templates with different sequences at the first position, based on differences of sequencing signals exhibited during base extension. This step includes: determining the relative numbers of the nucleic acid templates with different sequences and signal intensities thereof, based on the number and frequency of channels with sequencing signals, as well as the intensities of respective sequencing signals, acquired from the first position during at least one cycle of base extension.

In a first implementation, n=2, i.e., the plurality of nucleic acid molecules at the first position include 2 types of nucleic acid templates with different sequences. Correspondingly, the plurality of nucleic acid molecules include the primary nucleic acid molecule and the secondary nucleic acid molecule. At this point, in this step, when subjecting the nucleic acid molecules to base extension, the acquired sequencing signals from the respective channels include two sequencing signals with different intensities, namely the first sequencing signal with the highest signal intensity and the second sequencing signal with the second-highest signal intensity. Exemplarily, during a cycle of base extension, the sequencing signals from respective channels include a sequencing signal of base A and a sequencing signal of base T, and the ratio of the intensity of the sequencing signal of base A to the intensity of the sequencing signal of base T is approximately 2:1. Furthermore, the number of channels with sequencing signals and the intensities of the sequencing signals are reproduced over multiple cycles of base extension. Therefore, it can be concluded from the sequencing signals displayed by the signal channels that: the plurality of nucleic acid molecules at the first position include 2 types of nucleic acid templates with different sequences, and the ratio of molecule numbers of the two types of nucleic acid templates is approximately 2:1.

In a second implementation, n=3, i.e., the plurality of nucleic acid molecules at the first position include 3 types of nucleic acid templates with different sequences. Correspondingly, the plurality of nucleic acid molecules include a first nucleic acid molecule, a second nucleic acid molecule, and a third nucleic acid molecule. At this point, in this step, when subjecting the nucleic acid molecules to base extension, the acquired sequencing signals from the respective channels include three sequencing signals with different intensities, corresponding to the first nucleic acid molecule, the second nucleic acid molecule, and the third nucleic acid molecules. The sequencing signals include the first sequencing signal, the second sequencing signal, and the third sequencing signal with sequentially decreasing signal intensities. Exemplarily, during one cycle of base extension, the sequencing signals from respective channels include a sequencing signal of base A, a sequencing signal of base C, and a sequencing signal of base T, and the ratio of the intensities of the sequencing signal of base A, the intensity of the sequencing signal of base C, and the intensity of the sequencing signal of base T is approximately 10:7:3. Furthermore, the number of channels with sequencing signals and the intensities of the sequencing signals are reproduced over multiple cycles of base extension. Therefore, it can be concluded from the sequencing signals displayed by the signal channels that: the plurality of nucleic acid molecules at the first position include 3 types of nucleic acid templates with different sequences, and the ratio of the molecule numbers of the three types of nucleic acid templates is approximately 10:7:3.

In a third implementation, n=4, i.e., the plurality of nucleic acid molecules at the first position include 4 types of nucleic acid templates with different sequences. Correspondingly, the plurality of nucleic acid molecules include a first nucleic acid molecule, a second nucleic acid molecule, a third nucleic acid molecule, and a fourth nucleic acid molecule. At this point, in this step, when subjecting the nucleic acid molecules to base extension, the acquired sequencing signals from the respective channels include four sequencing signals with different intensities, corresponding to the first nucleic acid molecule, the second nucleic acid molecules, the third nucleic acid molecule, and the fourth nucleic acid molecule. The sequencing signals include the first sequencing signal, the second sequencing signal, the third sequencing signal, and the fourth sequencing signal with sequentially decreasing signal intensities. Exemplarily, during a cycle of base extension, the sequencing signals from respective channels include a sequencing signal of base A, a sequencing signal of base C, and a sequencing signal of base T, and the ratio of their intensities is approximately 13:7:3. During another cycle of base extension, the sequencing signals from respective channels include a sequencing signal of base A, a sequencing signal of for base G, a sequencing signal of for base C, and a sequencing signal of base T, and the ratio of their intensities is approximately 10:7:5:3. It can be concluded from the sequencing signals displayed by the signal channels that: the plurality of nucleic acid molecules at the first position include 4 types of nucleic acid templates with different sequences, and the ratio of the molecule numbers of the four types of nucleic acid templates are approximately 10:7:5:3.In this case, because for two or more of the four types of nucleic acid templates, the same type of base or nucleotide are introduced during one or more cycles of base extension, the sequencing signals they generate in the corresponding base channel will be superimposed. However, the relative proportion of the molecule numbers of the respective nucleic acid templates may still be obtained, according to the types of sequencing signals and their intensities obtained through multiple cycles of base extension.

According to embodiments of the present disclosure, because there are some interfering signals inevitably in the sequencing signals generated during the base extension process, sequencing signals with brightness great than or equal to 10% are selected from respective channels, with a standard brightness as 100% in the step of acquiring sequencing signals from respective channels, to increase the sequencing accuracy. In other words, during base extension, when acquiring sequencing signals from respective channels, the intensities of the sequencing signals from respective channels are evaluated at first; then according to the criterion of "selecting sequencing signals with brightness great than or equal to 10% from respective channels, with a standard brightness as 100%", sequencing signals from channels meeting the requirement are screened for the next step of base calling.

For example, the case of a first position containing two nucleic acid templates are simply illustrated. If, in the sequencing signals obtained from four channels, such as in the images generated by multiple cycles of base extension, when the signal collection is performed, there are always two channels with sequencing signals at a immobilized position or sequencing signals detected twice in a same channel, where the signal intensities of the two sequencing signals is in a relatively constant ratio of, e.g., 7:3, it can be determined that the copy number of the two nucleic acid templates at the position is about 7:3 according to the signal intensities. The nucleic acid template with the high content may be named the first nucleic acid template and the nucleic acid template with the low content may be named the second nucleic acid template, for ease of description.

It should be understood that the intensity or signal intensity referenced in this step may be a raw intensity corresponding to fluorescence images or a corrected signal intensity. As previously mentioned, the correction may include crosstalk correction and/or phase imbalance correction.

(2) sorting the respective sequencing signals in each cycle of base extension in a descending or ascending order, based on the relative signal intensities of the respective sequencing signals at a position corresponding to the first position during each cycle of base extension.

In a cycle of sequencing reaction, the n types of nucleic acid templates with different sequences at the first position may be exhibit as being with sequencing signals in different channels, or may be detected multiple times in the same channel during a signal collection. Because the molecule numbers (e.g., copy numbers) of the n types of nucleic acid templates with different sequences at the same position are different, the intensities of these sequencing signals will be different. The sequencing signals may be ranked based on their intensity differences. For example, at the first position containing two nucleic acid templates, if there are different nucleotides introduced for the primary nucleic acid molecule and the secondary nucleic acid molecule in a cycle of sequencing reaction, then in that cycle of sequencing reaction, there will be bright speckles at the first position in two different channels, or bright speckles detected twice at the first position in the same channel during multiple signal collection. Furthermore, due to the different molecule numbers of the two types of nucleic acid molecules, the sequencing signal intensities in the two channels will be different.

Taking fluorescence intensity as an example, at the first position containing two nucleic acid molecules, i.e. the primary nucleic acid molecule and the secondary nucleic acid molecules with a ratio of molecule number≈7:3, in a fluorescence image collected during a cycle of base extension, both the channel of base A and the channel of base T exhibit fluorescence signals, respectively, with a ratio of signal intensities in 7:3. At this point, sequencing signals from the two channels are ranked according to the fluorescence signal intensities in the descending or ascending order. For example, in the case of descending order, the sequencing signals of respective channels are ranked as the first sequencing signal and the second sequencing signal.

It should be understood that in this step, by ranking respective sequencing signals in each cycles of sequencing reaction, base types corresponding to signal intensity levels can be identified. Continuing with the previous example, in the fluorescence image collected during a cycle of base extension, both the channel of base A and the channel of base T exhibit fluorescence signals, respectively, with a ratio of signal intensities in 7:3. At this point, the base type corresponding to the first sequencing signal is base A, indicating the base introduced to the first nucleic acid molecule is base A; the base type corresponding to the second sequencing signal is base T, indicating the base introduced to the second nucleic acid molecule is base T.

It should be understood that in some embodiments, there is a case that the types of nucleotides, which are introduced to the n types of nucleic acid templates with different sequences at the same first position in the same cycle of base extension, are the same. At this point, the first position will exhibit a sequencing signal in one channel; alternatively, this position exhibits a sequencing signal in one channel that is significantly higher than in other channels, allowing the determination that only one type of nucleotide or nucleotide analog is introduced at this position during this cycle of sequencing reaction based on this signal intensity.

(3) Connecting in series, based on the order of the signal intensities in each cycle of base extension, the sequencing signals, and determining the sequencing result of the at least two types of nucleic acid templates.

In an embodiment, connecting in series, based on the ranking result, the sequencing signals during each cycle of base extension, and determining the sequencing result of the at least two types of nucleic acid templates, include:

determining the sequencing signals with the same rank during each cycle of base extension, based on the ranking result;
defining the sequencing signals with the same rank as sequencing signals generated by a same type of nucleic acid template;
connecting in series the sequencing signals of the same type of nucleic acid template during each cycle of base extension; and determining the sequencing result of the at least two types of nucleic acid templates based on the sequencing signals connected in series.

Accordingly, after ranking the sequencing signals obtained in each cycle of sequencing reaction, the base types corresponding to the base channel with the highest-ranked sequencing signal intensity in each cycle are connected in series sequentially, thereby obtaining the sequence of the nucleic acid template with the highest molecule number at the first position. By analogy, according to the descending or ascending order ranked by the intensities of the sequencing signal, sequences of nucleic acid templates corresponding to decreasing or increasing molecule numbers may be obtained sequentially.

Exemplarily, taking four-color sequencing on a nanowell array chip as an example, the process of identifying two sequences from a first position containing two nucleic acid templates with different sequences is illustrated. In principle, there may be dual-molecule signal spot formed by amplification of two DNA fragments in a nanowell of the array chip. Due to differences in amplification initiation timing and amplification efficiency, the ratio of amplification number between these two DNA fragments is 7:3. Taking four channels as an example, fluorescence images of four base channels are obtained during each cycle of base extension, enabling to extract four grayscale values from the images. Further, fluorescence intensities in the images may be subjected to the crosstalk correction and/or phase imbalance correction. If, in a cycle of sequencing reaction, the base signals of these two nucleic acid molecules are different, e.g., A and C respectively, then the ratio of intensities of the four bases A:C:G:T in the image would be 7:3:0:0, assuming the sum of the respective proportions of the 4-channel signals is 10. Accordingly, base A and base C are identified. After proceeding to the next cycle of sequencing reaction, if the bases introduced to both nucleic acid molecules are T, the ratio of intensities of the four bases in the image would be 0:0:0:10, indicating that both bases introduced in this cycle are T. In each cycle of reaction, the base with the highest intensity and the base with the second-highest intensity in the image are identified and ranked according to their brightness/darkness pattern, then the base with the highest intensity is connected in series with the base with the highest intensity, and the base with the second-highest intensity is connected in series with the base with the second-highest intensity, thereby obtaining the sequences of the two nucleic acid molecules. In this way, output of two sequences from one nanowell is achieved, i.e., the identification of dual nucleic acid molecules at a single spot is achieved.

In some embodiments, the method further includes: determining the nucleic acid sequence of the target subject based on the sequencing result.

The inventors of the present disclosure have experimentally verified the technical concept that sequencing signals can be effectively distinguished utilizing intensity differences after synchronous sequencing of different nucleic acid templates. In this way, sequencing throughput can be greatly increased. By employing the sequencing method, sequencing signals can be effectively classified based on different intensities, thereby achieving the effective utilization of multi-molecule signal spots and improving the sequencing throughput.

The solution of the present invention will be explained below with reference to examples. It would be appreciated by those skilled in the art that the following examples are explanatory, and cannot be construed to limit the scope of the present disclosure. If the specific technology or conditions are not specified in the examples, a step will be performed in accordance with the techniques or conditions described in the literature in the art or in accordance with the product instructions. If the manufacturers of reagents or instruments are not specified, the reagents or instruments may be commercially available.

**Example 1**

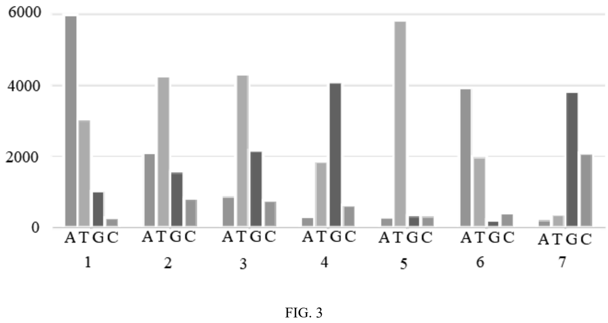

Sequencing was performed on a sequencing chip using a sequencer. Collected sequencing image data obtained from multiple consecutive cycles of base extension (cycles) were analyzed, thereby retaining sequencing signals with intensities ≥10% of the standard intensity. Image data from seven consecutive cycles of base extension at a dual-signal spot were taken as an example for illustration, as shown in FIG. 3:

In the fluorescence image obtained during the first cycle of base extension, the results showed that the ratio of intensities of the four bases corresponding to A, T, G, C was 6:3:0:0. Therefore, it was identified that the ratio of sequencing signal intensities between base A and base T was 2:1;
In the fluorescence image obtained during the second cycle of base extension, the results showed that the ratio of intensities of the four bases corresponding to A, T, G, C was 2:4:0:0. Therefore, it was identified that the ratio of sequencing signal intensities between base T and base A was 2:1.

In the fluorescence image obtained during the third cycle of base extension, the results showed that the ratio of intensities of the four bases corresponding to A, T, G, C was 0:4:2:0. Therefore, it was identified that the ratio of sequencing signal intensities between base T and base G was 2:1.

In the fluorescence image obtained during the fourth cycle of base extension, the results showed that the ratio of intensities of the four bases corresponding to A, T, G, C was 0:2:4:0. Therefore, it was identified that the ratio of sequencing signal intensities between base G and base T was 2:1.

In the fluorescence image obtained during the fifth cycle of base extension, the results showed that the ratio of intensities of the four bases corresponding to A, T, G, C was 0:6:0:0. Therefore, it was identified that both bases introduced during this cycle of sequencing reaction were T.

In the fluorescence image obtained during the sixth cycle of base extension, the results showed that the ratio of intensities of the four bases corresponding to A, T, G, C was 4:0:2:0. Therefore, it was identified that the ratio of sequencing signal intensities between base A and base G was 2:1.

In the fluorescence image obtained during the seventh cycle of base extension, the results showed that the ratio of intensities of the four bases corresponding to A, T, G, C was 0:0:4:2. Therefore, it was identified that the ratio of sequencing signal intensities between base G and base C was 2:1.

It was known from the fluorescence images obtained in the above cycles of base extension that, the dual-signal position contained two nucleic acid templates. In each cycle of base extension, the ratio between sequencing signals of the two nucleic acid templates were approximately 2:1. Thus, the molecule numbers of the two nucleic acid templates were approximately 2:1. The nucleic acid template with higher molecule numbers generated sequencing signals with higher intensities in the images of base extension. By connecting in series the base types identified by the sequencing signals with higher intensities in the images obtained during each cycle of base extension; similarly, by connecting in series the base types identified by the sequencing signals with higher intensities in the images obtained during each cycle of base extension, the sequences of the two nucleic acid templates were obtained as: ATTGTAG and TAGTTGC, respectively.

Thus, identification of dual nucleic acid molecules within a single spot was achieved.

Reference throughout this specification to "an embodiment", "some embodiments", "one embodiment", "another example", "an example", "a specific example" or "some examples" means that a particular feature, structure, material, or characteristic described in connection with the embodiment or example is included in at least one embodiment or example of the present disclosure. Thus, the appearances of the phrases such as "in some embodiments", "in one embodiment", "in an embodiment", "in another example", "in an example", "in a specific example" or "in some examples", in various places throughout this specification are not necessarily referring to the same embodiment or example of the present disclosure. Furthermore, the particular features, structures, materials, or characteristics may be combined in any suitable manner in one or more embodiments or examples.

Although explanatory embodiments have been shown and described, it would be appreciated by those skilled in the art that the above embodiments cannot be construed to limit the present disclosure, and changes, alternatives, and modifications can be made in the embodiments without departing from spirit, principles and scope of the present disclosure.

**Claims**

1. A sequencing method, comprising:

   providing a solid support having at least one first position at which a plurality of nucleic acid molecules are immobilized, the plurality of nucleic acid molecules having n different nucleic acid templates with different sequences, wherein n is an integer from 2 to 4;
   performing multiple cycles of base extension on the nucleic acid molecules and acquiring sequencing signals from respective channels during the base extension, wherein in the one or more cycles of base extension, the sequencing signals at the first position comprise at least a first sequencing signal with the highest signal intensity and a second sequencing signal with the second-highest signal intensity; and
   assigning the sequencing signals and determining a sequencing result of at least two types of nucleic acid templates, based on differences in the signal intensities of the respective sequencing signals.

2. The sequencing method of claim 1, wherein the signal intensities of the sequencing signals generated by the n types of nucleic acid templates with different sequences are different in each cycle of base extension.

3. The sequencing method of claim 1 or 2, wherein n=2, and the sequencing signals comprise the first sequencing signal with the highest signal intensity and the second sequencing signal with the second-highest signal intensity

4. The sequencing method of claim 1 or 2, wherein n=3, and the sequencing signals comprise the first sequencing signal, the second sequencing signal, and a third sequencing signal with sequentially decreasing signal intensities.

5. The sequencing method of claim 1 or 2, wherein n=4, and the sequencing signals comprise the first sequencing signal, the second sequencing signal, a third sequencing signal, and a fourth sequencing signal with sequentially decreasing signal intensities.

6. The sequencing method of any one of claims 1 to 5, for any two sequencing signals whose intensities are adjacent in magnitude, the signal intensity of a higher intensity signal is at least 1.2 times, preferably at least 1.5 times, and more preferably 1.5 to 10 times, the signal intensity of a lower intensity signal among the sequencing signals ranking in a descending or ascending order of signal intensities.

7. The sequencing method of any one of claims 1 to 5, wherein acquiring the sequencing signals from the respective channels comprises:
selecting sequencing signals with brightness ≥10% from respective channels, with a standard brightness as 100%.

8. The sequencing method of any one of claims 1 to 7, wherein assigning the sequencing signals and determining the sequencing result of at least two types of nucleic acid templates, based on differences in the signal intensities of the respective sequencing signals comprise:

determining a number of the nucleic acid templates and relative signal intensities corresponding to respective nucleic acid templates, based on types and intensities of respective sequencing signals acquired from the first position during at least one cycle of base extension; sorting the respective sequencing signals in each cycle of base extension in a descending or ascending order according to relative signal intensities of the respective sequencing signals at a position corresponding to the first position during each cycle of base extension; and connecting in series, based on the order of the signal intensities in each cycle of base extension, the sequencing signals and determining the sequencing result of the at least two types of nucleic acid templates.

9. The sequencing method of claim 8, wherein connecting in series, based on the order of the signal intensities in each cycle of base extension, the sequencing signals, and determining the sequencing result of the at least two types of nucleic acid templates comprise:

determining, based on the order, sequencing signals with the same rank in each cycle of base extension;
defining the sequencing signals with the same rank as sequencing signals generated by a same type of nucleic acid template;
connecting in series the sequencing signals of the same type of nucleic acid template during each cycle of base extension, and determining the sequencing result of the at least two types of nucleic acid templates based on the sequencing signals connected in series.

10. The sequencing method of any one of claims 1 to 9, wherein the signal intensities of the sequencing signals are subjected to a correction.

11. The sequencing method of claim 10, wherein the correction comprises at least one of an inter-channel crosstalk correction or an intra-channel crosstalk correction.

12. The sequencing method of any one of claims 1 to 11, wherein the sequencing signal is an optical signal.

13. The sequencing method of any one of claims 1 to 11, wherein the n nucleic acid templates with different sequences at the first position are different from each other in molecule numbers.

14. The sequencing method of claim 13, for any two sequencing signals whose molecule numbers are adjacent in magnitude, the molecule number of the nucleic acid templates with a higher molecule number is at least 1.2 times, preferably at least 1.5 times, and more preferably 1.5 to 10 times, the molecule number of the nucleic acid templates with a lower molecule number between two adjacent nucleic acid templates among the nucleic acid templates ranking in a descending or ascending order.

15. The sequencing method of any one of claims 1 to 14, wherein at least two types of nucleic acid templates among the n types of nucleic acid templates with different sequences generate different sequencing signals in at least one cycle of base extension.

16. The sequencing method of any one of claims 1 to 15, wherein the solid support is further provided with a second position, at which a plurality of nucleic acid molecules are immobilized, the plurality of nucleic acid molecules having identical sequences.

17. The sequencing method of any one of claims 1 to 15, wherein the plurality of nucleic acid molecules are immobilized on the surface of the position through bridge amplification.

18. The sequencing method of claim 17, wherein the plurality of nucleic acid molecules at the first position are formed by performing bridge amplification on different nucleic acid templates.

19. The sequencing method of any one of claims 1 to 18, wherein

the is a substrate with an array of microwells, and each position corresponds to one microwell; or
the solid support is a planar substrate, and a surface of the planar substrate is provided with a plurality of surface-treated positions, and each position corresponds to one surface-treated position.

20. The sequencing method of any one of claims 1 to 18, further comprising:

constructing sequencing library molecules based on a target subject, thereby enabling the plurality of nucleic acid molecules to be obtained by performing amplification on the sequencing library molecules on the surface of the solid support.

21. The sequencing method of claim 20, further comprising: determining a nucleic acid sequence of the target subject based on the sequencing result.

FIG. 1

FIG. 2

FIG. 3

## INTERNATIONAL SEARCH REPORT

| International application No. |
|---|
| **PCT/CN2024/075583** |

### A. CLASSIFICATION OF SUBJECT MATTER

C12Q 1/6874(2018.01)i;  C12Q 1/6806(2018.01)i;  C40B 50/06(2006.01)i

According to International Patent Classification (IPC) or to both national classification and IPC

### B. FIELDS SEARCHED

Minimum documentation searched (classification system followed by classification symbols)

IPC: C12Q,  C40B

Documentation searched other than minimum documentation to the extent that such documents are included in the fields searched

Electronic data base consulted during the international search (name of data base and, where practicable, search terms used)

CNABS, VEN, CNKI, CNTXT, USTXT, EPTXT, PUBMED, ISI WEB OF SCIENCE: 深圳市真迈生物科技有限公司, 李林森, 测序, 高通量, 核酸, 固相载体, 通道, 信号, 差异, sequencing, nucleic acids, solid support, channel, signal, difference, gap, compare

### C. DOCUMENTS CONSIDERED TO BE RELEVANT

| Category* | Citation of document, with indication, where appropriate, of the relevant passages | Relevant to claim No. |
|---|---|---|
| Y | US 2018282800 A1 (BGI SHENZHEN) 04 October 2018 (2018-10-04)<br>claims 1-29, and description, paragraphs 68 and 92 | 1-21 |
| Y | US 2019352707 A1 (ULTIMA GENOMICS, INC.) 21 November 2019 (2019-11-21)<br>claims 1-53 | 1-21 |
| A | WO 2022247555 A1 (GENEMIND BIOSCIENCES CO., LTD.) 01 December 2022<br>(2022-12-01)<br>claims 1-29 | 1-21 |
| A | CN 114196744 A (CYGNUS BIOSCIENCES (BEIJING) CO., LTD.) 18 March 2022<br>(2022-03-18)<br>claims 1-8 | 1-21 |
| A | CN 114550818 A (CYGNUS BIOSCIENCES (BEIJING) CO., LTD.) 27 May 2022<br>(2022-05-27)<br>claims 1-11 | 1-21 |
| A | US 2022002798 A1 (BGI SHENZHEN) 06 January 2022 (2022-01-06)<br>claims 1-22 | 1-21 |

☑ Further documents are listed in the continuation of Box C.  ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **20 May 2024** | **23 May 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2024/075583** |

| C. | DOCUMENTS CONSIDERED TO BE RELEVANT | |
| --- | --- | --- |
| Category* | Citation of document, with indication, where appropriate, of the relevant passages | Relevant to claim No. |
| A | CN 102831330 A (BEIJING NOVOGENE BIOINFORMATICS TECHNOLOGY CO., LTD.) 19 December 2012 (2012-12-19) claims 1-15 | 1-21 |

Form PCT/ISA/210 (second sheet) (July 2022)

<table>
<tr><td colspan="2">INTERNATIONAL SEARCH REPORT<br>Information on patent family members</td><td>International application No.<br>**PCT/CN2024/075583**</td></tr>
</table>

| Patent document cited in search report | | | Publication date (day/month/year) | Patent family member(s) | | | Publication date (day/month/year) |
|---|---|---|---|---|---|---|---|
| US | 2018282800 | A1 | 04 October 2018 | WO | 2016077313 | A1 | 19 May 2016 |
| | | | | US | 10626455 | B2 | 21 April 2020 |
| | | | | DK | 3218519 | T3 | 21 December 2020 |
| | | | | EP | 3218519 | A1 | 20 September 2017 |
| | | | | EP | 3218519 | A4 | 25 July 2018 |
| | | | | EP | 3218519 | B1 | 02 December 2020 |
| US | 2019352707 | A1 | 21 November 2019 | US | 2023313287 | A1 | 05 October 2023 |
| | | | | WO | 2018064297 | A1 | 05 April 2018 |
| WO | 2022247555 | A1 | 01 December 2022 | EP | 4350007 | A1 | 10 April 2024 |
| CN | 114196744 | A | 18 March 2022 | None | | | |
| CN | 114550818 | A | 27 May 2022 | None | | | |
| US | 2022002798 | A1 | 06 January 2022 | WO | 2020113581 | A1 | 11 June 2020 |
| CN | 102831330 | A | 19 December 2012 | None | | | |

Form PCT/ISA/210 (patent family annex) (July 2022)